# EUROPÄISCHE PATENTSCHRIFT

(11) **EP 2 168 985 B1**
(45) Veröffentlichungstag und Bekanntmachung des Hinweises auf die Patenterteilung: **27.08.2014**
(21) Anmeldenummer: 09011791.2
(22) Anmeldetag: 16.09.2009
(51) Int. Cl.: C07K 16/36, C12N 9/74, C07K 16/42, G01N 33/86

(54) **Antikörper zur Bestimmung des Prothrombin-Fragments F2/F1+2 in einem homogenen Immunoassay**
Antibodies for determining the prothrombin fragment F2/F1+2 in a homogenous immunoassay
Anticorps destinés à la détermination du fragment de prothrombine F2/F1+2 dans un test d'immunité homogène

(30) Priorität: 30.09.2008 DE 102008049601
(43) Veröffentlichungstag der Anmeldung: 31.03.2010
(73) Patentinhaber: Siemens Healthcare Diagnostics Products GmbH, 35041 Marburg (DE)
(72) Erfinder: Althaus, Harald, 35083 Wetter (DE); Barten, Roland, 35039 Marburg (DE); Ehm, Matthias, 35094 Lahntal (DE); Fischer, Bodo, 35039 Marburg (DE); Teigelkamp, Stefan, verstorben (DE)

(56) Entgegenhaltungen:
- EP-A1- 0 475 784
- US-A- 5 830 681
- US-A1- 2003 219 845

## Beschreibung

Die vorliegende Erfindung liegt auf- dem Gebiet der Gerinnungsdiagnostik und betrifft Antikörper, die spezifisch an einen Komplex aus F2/F1+2 und einem F2/F1+2-spezifischen Bindungspartner binden, sowie deren Herstellung und deren Verwendung in Verfahren zur Bestimmung von F2/F1+2.

Für die klinische Beurteilung des Status des Blutgerinnungssystems eines Patienten stehen eine Vielzahl diagnostisch relevanter Parameter zur Verfügung. Insbesondere der quantitative Nachweis sogenannter Aktivierungsmarker der Gerinnung ermöglicht die Beurteilung des Gerinnungs- oder Fibrinolyseprozesses. Die Bestimmung des Prothrombin-Fragments F2/F1+2 in einer Patientenprobe ermöglicht den Nachweis oder Ausschluss einer gesteigerten Thrombinbildung in vivo und wird zum Beispiel zur Erfassung der intravasalen Thrombinbildung bei einer Verbrauchskoagulopathie, bei akuten venösen Thromboembolien oder bei arteriellen Gefäßverschlüssen (Myokardinfarkt, Hirninfarkt) oder zur Überwachung von Antikoagulanzientherapien eingesetzt.

Prothrombin ist das Proenzym von Thrombin, dem zentralen Enzym der Blutgerinnungskaskade. Das Prothrombin-Protein ist modular aufgebaut und besteht aus einem N-terminalen F1+2-Anteil und einem C-terminalen Thrombin-Anteil. Durch die proteolytische Aktivität des Faktors Xa wird Prothrombin gespalten, so dass je Prothrombin-Molekül (70 kD) ein Thrombin-Molekül (30 kD) unter Freisetzung eines Prothrombin-Fragments F1+2 (35-37 kD) entsteht. Durch die autokatalytische Spaltung von Prothrombin durch Thrombin kann es des weiteren zur Spaltung der Fragmente F1 (23 kDa) und F2 (14 kDa) kommen. Da Thrombin in freier Form im Blut nicht vorkommt, sondern unmittelbar im Anschluss an seine Bildung an Inhibitoren und an Fibrin gebunden wird, muss das Ausmaß der Thrombinbildung indirekt erfasst werden, z. B. durch die Bestimmung der Aktivierungsmarker F1+2 und/oder F2. Eine Möglichkeit zum Nachweis oder zum Ausschluss einer gesteigerten Thrombinbildung in vivo ist demnach die Bestimmung der F2/F1+2-Konzentration im Plasma.

Generell tritt bei F2/F1+2-Testen die Schwierigkeit auf, dass Prothrombin in der Probe im Vergleich zu F2/F1+2 in 1 000 bis 10 000fachem molarem Überschuss vorliegt. Der immunologische Nachweis von F2/F1+2 in Gegenwart von Prothrombin wird dadurch erschwert, dass die freigesetzten Prothrombin-Fragmente F2/F1+2 gegenüber intaktem, ungespaltenem Prothrombin nur über ein einziges F2/F1+2-spezifisches, antigenes Epitop verfügen und zwar den nach der Abspaltung von Thrombin entstehenden Carboxyterminus des F2/F1+2-Peptids (Neoepitop). Ausschließlich Antikörper, die gegen den carboxyterminalen Bereich des F2/F1+2-Peptids gerichtet sind, sind in der Lage spezifisch an die Prothrombin-Fragmente F2/F1+2 zu binden, ohne gleichzeitig eine Spezifität für intaktes Prothrombin aufzuweisen. Alle anderen antigenen Determinanten des F2/F1+2-Fragmentes sind ebenso spezifisch für intaktes Prothrombin.

Die Herstellung spezifischer F2/F1+2-Antikörper, die nicht an Prothrombin binden, ist z. B. in EP 303 983 A2 (Pelzer et al.), in US 5,830,681 (Hursting et al.) oder in US 2003/0219845 A1 (Ruiz et al.) beschrieben. Für die Spezifität der anti-F2/F1+2-Antikörper ist wichtig, dass diese an ein Epitop binden, welches zumindest die vier carboxyterminalen Aminosäuren der F2/F1+2-Fragmente (Ile-Glu-Gly-Arg-OH) enthält. Da in der Regel zur Bestimmung der F2/F1+2-Konzentration Sandwich-Immunoassays eingesetzt werden, werden zwei anti-F2/F1+2-Antikörper benötigt. In EP 1 541 676 A1 (Teigelkamp et al.) sind Antikörper beschrieben, die an ein Epitop auf der N-terminalen Hälfte des F2-Fragments und damit auch an intaktes Prothrombin binden, sich jedoch zur Verwendung als Sekundärantikörper in Kombination mit den F2/F1+2-Neoepitop-spezifischen Primärantikörpern in einem Sandwich-Immunoassay besonders eignen.

Die bekannten Antikörper eignen sich zur Bestimmung der F2/F1+2-Konzentration in Plasmaproben in heterogenen Immunoassays, bevorzugt in Form eines Sandwich-ELISA-Verfahrens. Dazu werden die F2/F1+2-Neoepitop-spezifischen Antikörper ("Primärantikörper") an eine Festphase gekoppelt und mit der Probe inkubiert, so dass die F2/F1+2-Peptide an die immobilisierten Antikörper binden können. Durch einen Waschschritt werden ungebundene Proteine, insbesondere Prothrombin, entfernt, bevor durch Zugabe einer Antikörperlösung, der zweite, F2/F1+2- und Prothrombin-bindende Antikörper ("Sekundärantikörper") appliziert wird. In der Regel ist dieser Zweitantikörper mit einer signalbildenden Komponente assoziiert, die eine Quantifizierung der-F2/F1+2-Konzentration erlaubt.

Der Aufbau eines direkten, homogenen Sandwich-Immunoassays ohne Wasch-oder Trennschritte ist mit den aus dem Stand der Technik bekannten monoklonalen Antikörpern jedoch nur eingeschränkt möglich, da der F2/F1+2- und Prothrombin-bindende Sekundärantikörper im homogenen Testaufbau zum Großteil durch das in der Probe enthaltene Prothrombin gebunden wird und somit nicht mehr für die Sandwich-Bildung mit dem Analyten F2/F1+2, welche für die Signalgenerierung unerlässlich ist, zur Verfügung steht.

Der vorliegenden Erfindung lag also die Aufgabe zugrunde, Mittel bereitzustellen, die es ermöglichen, F2/F1+2 in Proben, die Prothrombin im Überschuss enthalten, spezifisch in einem homogenen Bindungstest zu bestimmen.

Die Lösung der Aufgabe besteht in der Bereitstellung der in den Ansprüchen beschriebenen erfindungsgemäßen Mittel und Verfahren.

Insbesondere wird die Aufgabe dadurch gelöst, dass ein monoklonaler Antikörper bereitgestellt wird, der spezifisch an einen Immunkomplex bindet, welcher Prothrombin-Fragment F2/F1+2, an welches ein Antikörper oder ein Antikörperfragment mit Spezifität für das carboxyterminale Neoepitop des Prothrombin-Fragmentes F2/F1+2 gebunden ist, enthält und wobei der Antikörper aber nicht an das Prothrombin-Fragment F1+2 oder F2 alleine und nicht an den Antikörper oder das Antikörperfragment mit Spezifität für das carboxyterminale Neoepitop des Prothrombin-Fragmentes F2/F1+2 alleine bindet.

Antikörper mit Spezifität für lmmunkomplexe, welche aus einem Analyten, an den ein analytspezifischer Bindungspartner, z. B. ein Primärantikörper oder ein Primärantikörperfragment gebunden ist, bestehen, sind prinzipiell bekannt, z. B. aus EP 264 219 A2, EP 475 784 A1, WO 85/04422 A1 oder WO 87/07147. In bezug auf die vorliegende Erfindung wurde jedoch überraschenderweise festgestellt, dass zur Gewinnung eines erfindungsgemäßen Antikörpers mit Spezifität für einen Prothrombin-Fragment F2/F1+2-Bindungspartner-Immunkomplex, ein Immunkomplex als Immunisierungsantigen verwendet werden muss, der ein Peptid enthält, das zumindest das vollständige Prothrombin-Fragment F2 umfasst, an welches ein Antikörperfragment mit Spezifität für das carboxyterminale Neoepitop des Prothrombin-Fragmentes F2/F1+2 gebunden ist. Mit Immunkomplexen, die nur ein Fragment des F2-Prothrombin-Fragments enthielten sowie mit Immunkomplexen, die einen vollständigen Antikörper mit Spezifität für das carboxyterminale Neoepitop des Prothrombin-Fragmentes F2/F1+2 enthielten, konnten keine Antikörper mit Spezifität für den Immunkomplex generiert werden.

Gegenstand der vorliegenden Erfindung ist ein isolierter, gereinigter Immunkomplex enthaltend ein Peptid, das zumindest das vollständige Prothrombin-Fragment F2 umfasst, an dessen Carboxyterminus ein Antikörperfragment mit Spezifität für das carboxyterminale Neoepitop des Prothrombin-Fragments F2/F1+2, welches zumindest die vier carboxyterminalen Aminosäuren Ile-Glu-Gly-Arg-OH enthält, gebunden ist und wobei der Immunkomplex an ein immunstimulatorisches Trägerprotein gekoppelt ist.

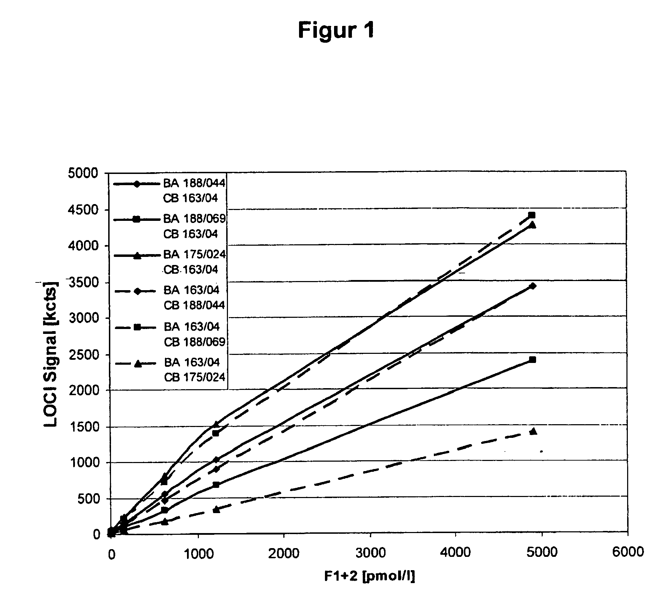

Ein Peptid, das zumindest das vollständige Prothrombin-Fragment F2 umfasst, d. h. die Aminosäurereste 156-273 von humanem Prothrombin (siehe z. B. Fig. 1 in Walz, D.A. (1977) Amino acid sequence of human prothrombin fragments 1 and 2. Proc. Natl. Acad. Sci. USA Vol. 74, No. 5, 1969-1972), und das das carboxyterminale Neoepitop des Prothrombin-Fragments F2/F1+2 aufweist, kann z. B. durch Inkubation einer Prothrombin-haltigen Lösung mit Faktor Xa bzw. durch Aktivierung der Blutgerinnungskaskade in einer Blutgerinnungsfaktor-haltigen Lösung, beispielsweise mit dem Schlangengift Russell's Viper Venom (RW), und anschließender chromatographischer Reinigung des F2-Fragments gewonnen werden. Alternativ kann das Prothrombin-Fragment F2 synthetisch durch chemische Synthese oder rekombinant durch Expression in einem prokaryotischen oder eukaryotischen Expressionssystem gewonnen werden.

Geeignet sind alle Peptide, die zumindest das vollständige Prothrombin-Fragment F2 inklusive des carboxyterminalen Neoepitops umfassen, also auch das vollständige- Prothrombin-Fragment F1+2 sowie die verschiedenen aminoterminal verkürzten F1+2-Fragmente.

Geeignete Antikörperfragmente mit Spezifität für das carboxyterminale Neoepitop des Prothrombin-Fragments F2/F1+2, wie z. B. Fab, Fab', F(ab')2 oder Fv-Fragmente, können beispielsweise rekombinant oder mittels enzymatischer Spaltung von Antikörpern, vorzugsweise von monoklonalen Antikörpern, gewonnen werden, wobei die Antikörper spezifisch sind für das carboxyterminale Neoepitop des Prothrombin-Fragments F2/F1+2 und nicht an Prothrombin binden. Derartige F2/F1+2-spezifische Antikörper, die nicht an Prothrombin binden, sind z. B. in EP 303 983 A2 (Pelzer et al.), in US 5,830,681 (Hursting et al.) oder in US 2003/0219845 A1 (Ruiz et al.) beschrieben. Die enzymatische Spaltung von monoklonalen Antikörpern kann beispielweise mittels Papain, Pepsin oder Ficin durchgeführt werden. Besonders bevorzugte Antikörperfragmente sind Fab-Fragmente, die z. B. mittels enzymatischer Spaltung durch Papain gewonnen werden können.

Ganz besonders bevorzugte Antikörperfragmente sind Fragmente, insbesondere Fab-Fragmente, des monoklonalen, F2/F1+2-Neoepitop-spezifischen Antikörpers 96-163/04, der gemäß der Lehre in US 5,830,681 (Hursting et al., insbesondere Examples I-VI) unter Verwendung eines synthetischen Peptids der Sequenz Cys-Gly-Ser-Asp-Arg-Ala-Ile-Glu-Gly-Arg-OH ("PF2") als Immunisierungsantigen hergestellt wurde und der von einer Hybridomazelllinie produziert wird, die am 03. Juni 2008 bei der DSMZ - Deutsche Sammlung von Mikroorganismen und Zellkulturen GmbH, Inhoffenstraße 7B, 38124 Braunschweig, Deutschland, unter der Eingangsnummer DSM ACC2911 hinterlegt wurde.

Zur Herstellung des erfindungsgemäßen Immunkomplexes werden das Peptid, das zumindest das vollständige Prothrombin-Fragment F2 umfasst und das Antikörperfragment mit Spezifität für das carboxyterminale Neoepitop des Prothrombin-Fragments F2/F1+2 zur Bildung des Immunkomplexes zusammengegeben und unter geeigneten Bedingungen inkubiert. Anschließend wird der Immunkomplex von den übrigen Reaktanden abgetrennt, d. h. isoliert und gereinigt, bevorzugt chromatographisch.

In einer bevorzugten Ausführungform des erfindungsgemäßen Immunkomplexes ist der Komplex chemisch quervernetzt, vorzugsweise durch Behandlung des Komplexes mit einem Aldehyd, wie z. B. Glutaraldehyd oder Formaldehyd.

Weiterhin ist der Immunkomplex an ein Trägerprotein, wie beispielsweise Keyhole Limpet Hämocyanin oder Ovalbumin gekoppelt. Die immunstimulatorische Wirkung derartiger Trägerproteine und Verfahren zu deren Kopplung sind im Stand der Technik bekannt.

Ein weiterer Gegenstand der vorliegenden Erfindung betrifft die Verwendung eines erfindungsgemäßen Immunkomplexes als Immunisierungsantigen in einem Verfahren zur Gewinnung monoklonaler Antikörper, die spezifisch den Komplex binden, aber nicht die einzelnen Komponenten des Komplexes. Verfahren zur Gewinnung monoklonaler Antikörper sind hinreichend bekannt, wie z. B. die Etablierung von Hybridomazellen mit anschließender Reinigung der sekretierten Antikörper.

Die Erfindung betrifft weiterhin monoklonale Antikörper, die sich dadurch auszeichnen, dass sie an einen Immunkomplex enthaltend ein Peptid, das zumindest das vollständige Prothrombin-Fragment F2 umfasst, an welches ein Antikörperfragment mit Spezifität für das carboxyterminale Neoepitop des Prothrombin-Fragments F2/F1+2 gebunden Ist, binden, aber nicht an das Prothrombin-Fragment F2 alleine und nicht an das Antikörperfragment mit Spezifität für das carboxyterminale Neoepitop des Prothrombin-Fragmentes F2/F1 +2 alleine.

Besonders bevorzugte Antikörper im Sinne der vorliegenden Erfindung sind Antikörper, die spezifisch an einen Immunkomplex binden, der das vollständige F2-Peptid enthält, an welches ein Fab-Fragment eines monoklonalen Antikörpers gebunden ist, der von der Hybridomazelllinie DSM ACC 2911 gebildet wird.

Derartige Antikörper werden z. B. von den Hybridomazelllinien 2006-175/024, 2006-188/044 oder 2006-188/069 produziert. Diese Hybridomazelllinien wurden am 03. Juni 2008 bei der DSMZ - Deutsche Sammlung von Mikroorganismen und Zellkulturen GmbH, Inhoffenstraße 7B, 38124 Braunschweig, Deutschland, unter den Eingangsnummern DSM ACC2912, DSM ACC2913 und DSM ACC2914 hinterlegt.

Gegenstand dieser Erfindung ist auch ein erfindungsgemäßer Antikörper, der mit einer Festphase und/oder einer Komponente eines signalbildenden Systems assoziiert ist.

Der Begriff "Festphase" im Sinne dieser Erfindung beinhaltet einen Gegenstand, der aus porösem und/oder nicht porösem, in der Regel wasserunlöslichem Material besteht und die unterschiedlichsten Formen aufweisen kann, wie z. B. Gefäß, Röhrchen, Mikrotitrationsplatte, Kugel, Mikropartikel, Stäbchen, Streifen, Filter- oder Chromatographiepapier etc. In der Regel ist die Oberfläche der Festphase hydrophil oder kann hydrophil gemacht werden. Die Festphase kann aus den unterschiedlichsten Materialien bestehen wie z. B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere wie z. B. Zellulose, Nitrozellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Keramik, Glas, Metalle, insbesondere Edelmetalle wie Gold und Silber; Magnetit, Mischungen oder Kombinationen derselben etc.

Die Festphase kann einen Überzug aus einer oder mehreren Schichten aufweisen, z. B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern, oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkender Agenzien.

Bei einem "signalbildenden System" kann es sich um eine oder mehrere Komponenten handeln, wobei es sich wenigstens bei einer Komponente um ein nachweisbares Label handelt. Als Label ist jedes Molekül zu verstehen, das selbst ein Signal produziert oder die Produktion eines Signals induzieren kann wie z. B. eine fluoreszierende Substanz, eine radioaktive Substanz, ein Enzym oder eine chemilumineszierende Substanz. Das Signal kann beispielsweise anhand der Enzymaktivität, der Lumineszenz, der Lichtabsorption, der Lichtstreuung, der ausgestrahlten elektromagnetischen oder radioaktiven Strahlung oder einer chemischen Reaktion nachgewiesen oder gemessen werden.

Ein Label vermag selbst ein nachweisbares Signal zu erzeugen, so dass keine weiteren Komponenten notwendig sind. Viele organische Moleküle absorbieren ultraviolettes und sichtbares Licht, wobei durch die Lichtabsorption übertragene Energie diese Moleküle in einen angeregten Energiezustand kommen können und die absorbierte Energie in Form von Licht einer anderen Wellenlänge als der des eingestrahlten Lichts abgeben. Wieder andere Label können direkt ein nachweisbares Signal erzeugen wie z. B. radioaktive Isotope oder Farbstoffe.

Wieder andere Label benötigen zur Signalerzeugung weitere Komponenten, d. h. das signalproduzierende System schließt in einem solchen Fall all die für die Signalbildung benötigten Komponenten mit ein wie z. B. Substrate, Coenzyme, Quencher, Beschleuniger, zusätzliche Enzyme, Substanzen die mit Enzymprodukten reagieren, Katalysatoren, Aktivatoren, Cofaktoren, Inhibitoren, Ionen etc.

Geeignete Label sind beispielsweise Enzyme einschließlich Meerrettichperoxidase, alkalische Phosphatase, Glukose-6-Phosphatdehydrogenase, Alkoholdehydrogenase, Glucoseoxidase, β -Galactosidase, Luciferase, Urease und Acetylcholinesterase; Farbstoffe; fluoreszierende Substanzen einschließlich Fluoresceinisothiocyanat, Rhodamin, Phycoerythrin, Phycocyanin, Ethidiumbromid, 5-Dimethylaminonaphthalen-1-sulfonylchlorid und fluoreszierende Chelate von seltenen Erden; chemilumineszierende Substanzen einschließlich Luminol, Isoluminol, Acridiniumverbindungen, Olefin, Enolether, Enamin, Arylvinylether, Dioxen, Arylimidazol, Lucigenin, Luciferin und Aequorin; Sensitizer einschließlich Eosin, 9,10-Dibromoanthracen, Methylen Blau, Porphyrin, Phthalocyanin, Chlorophyll, Rose Bengal; Coenzyme; Enzymsubstrate; radioaktive Isotope einschließlich ¹²⁵I, ¹³¹I, ¹⁴C, ³H, ³²P, ³⁵S, ¹⁴C, ⁵¹Cr, ⁵⁹Fe, ⁵⁷Co und ⁷⁵Se; Partikel einschließlich magnetische Partikel oder Partikel, bevorzugt Latexpartikel, die selbst beispielsweise mit Farbstoffen, Sensitizern, fluoreszierenden Substanzen, chemilumineszierenden Substanzen, Isotopen oder anderen nachweisbaren Labeln markiert sein können; Solpartikel einschließlich Gold- oder Silbersolen etc.

Ein signalbildendes System kann auch Komponenten umfassen, die bei räumlicher Nähe miteinander in eine nachweisbare Wechselwirkung treten können, z. B. in Form von Energiespendern und Energieempfängern wie beispielsweise Photosensitizer und chemolumineszierende Substanzen (siehe z. B. EP 515 194 A2), Photosensitizer und Fluorophore (WO 95/06877), radioaktives lod-125 und Fluorophore (Udenfriend, S. et al. (1985) Proc. Natl. Acad. Sci. 82: 8672-8676), Fluorophore und Fluorophore (Mathis, G. (1993) Clin. Chem. 39: 1953-1959) oder Fluorophore und Fluoreszenz-Quencher (US 3,996,345). Unter einer Wechselwirkung zwischen den Komponenten ist die direkte Übertragung von Energie zwischen den Komponenten, z. B. durch Licht-oder Elektronenstrahlung sowie über kurzlebige reaktive chemische Moleküle, miteingeschlossen. Ferner umfasst sind hiervon auch Vorgänge, bei denen die Aktivität einer Komponente durch eine oder mehrere andere inhibiert oder verstärkt wird, beispielsweise die Hemmung oder Steigerung der Enzymaktivität oder die Hemmung, Steigerung oder Veränderung (z. B. Wellenlängenverschiebung, Polarisation) des von der beeinflußten Komponente ausgesendeten elektromagnetischen Strahlung. Die Wechselwirkung zwischen den Komponenten umfasst auch Enzymkaskaden. In diesem Fall sind die Komponenten Enzyme, von denen mindestens eines das Substrat für ein anderes liefert, so dass eine maximale oder minimale Reaktionsgeschwindigkeit der gekoppelten Substratumsetzung resultiert.

Eine effektive Wechselwirkung zwischen den Komponenten findet in der Regel statt, wenn diese räumlich benachbart vorliegen, also z. B. innerhalb eines Abstandbereiches von wenigen Mikrometern, insbesondere innerhalb eines Abstandsbereiches von unter 600 nm, bevorzugt unter 400 nm, ganz besonders bevorzugt von unter 200 nm.

Mikropartikel werden häufig als Festphase und/oder als Label genutzt. Unter dem Begriff "Mikropartikel" sind im Sinne dieser Erfindung Teilchen zu verstehen, die einen ungefähren Durchmesser von wenigstens 20 nm und nicht-mehr als 20 µm aufweisen, üblicherweise zwischen 40 nm und 10 µm, bevorzugt zwischen 0,1 und 10 µm, besonders bevorzugt zwischen 0,1 und 5 µm, ganz besonders bevorzugt zwischen 0,15 und 2 µm. Die Mikropartikel können regelmäßig oder unregelmäßig geformt sein. Sie können Kugeln, Spheroide, Kugeln mit mehr oder weniger großen Kavitäten oder Poren darstellen. Die Mikropartikel können aus organischem, aus anorganischem Material oder aus einer Mischung oder Kombination von beiden bestehen. Sie können aus einem porösen oder nicht porösen, einem schwellfähigen oder nicht schwellfähigen Material bestehen. Prinzipiell können die Mikropartikel jegliche Dichte haben, bevorzugt sind jedoch Partikel mit einer Dichte, die der Dichte des Wassers nahe kommt wie etwa 0,7 bis etwa 1,5 g/ml. Die bevorzugten Mikropartikel sind in wässrigen Lösungen suspendierbar und möglichst lange suspensionsstabil. Sie mögen durchsichtig, teilweise durchsichtig oder undurchsichtig sein. Die Mikropartikel können aus mehreren Schichten bestehen wie beispielsweise die sogenannten "core-andshell" Partikel mit einem Kern und einer oder mehreren umhüllenden Schichten. Der Begriff Mikropartikel umfasst beispielsweise Farbstoffkristalle, Metallsolen, Silica-Partikel, Glaspartikel, Magnetpartikel, Polymerteilchen, Öltropfen, Lipidpartikel, Dextran, und Proteinaggregate. Bevorzugte Mikropartikel sind in wässrigen Lösungen suspendierbare und aus wasserunlöslichem Polymermaterial bestehende Partikel, insbesondere aus substituierten Polyethylenen. Ganz besonders bevorzugt sind Latexpartikel z. B. aus Polystyrol, Acrylsäurepolymeren, Methacrylsäurepolymeren, Acrylnitril-Polymeren, Acrylnitril-Butadien-Styrol, Polyvinylacetat-Acrylat, Polyvinylpyridin, Vinylchlorid-Acrylat. Von besonderem Interesse sind Latexpartikel mit reaktiven Gruppen an ihrer Oberfläche wie beispielsweise Carboxyl-, Amino- oder Aldehydgruppen, die eine kovalente Bindung z. B. von spezifischen Bindungspartnern an die Latexpartikel erlauben. Die Herstellung von Latexpartikeln ist beispielsweise in EP 80 614 A2, EP 227 054 A2 und EP 246 446 A2 beschrieben.

Der Begriff "assoziiert" ist breit zu verstehen und umfasst beispielsweise eine kovalente und eine nicht-kovalente Bindung, eine direkte und eine indirekte Bindung, die Adsorption an eine Oberfläche und den Einschluss in eine Vertiefung oder einen Hohlraum etc. Bei einer kovalenten Bindung sind die Antikörper über eine chemische Bindung an die Festphase oder an eine Komponente eines signalgebenden Systems gebunden. Beispiele für eine nicht-kovalente Bindung sind die Oberflächenadsorption, der Einschluss in Hohlräume oder die Bindung von zwei spezifischen Bindungspartnern. Neben einer direkten Bindung an die Festphase oder die Komponente eines signalgebenden Systems können die Antikörper auch indirekt über spezifische Wechselwirkung mit anderen spezifischen Bindungspartnern an die Festphase oder das Label gebunden sein (siehe z. B. EP 411 945 A2). Dies soll anhand von Beispielen näher illustriert werden: Der biotinylierte Antikörper kann über labelgebundenes Avidin an das Label gebunden werden, oder es kann ein Fluorescein-Antikörperkonjugat über festphasengebundene Anti-Fluorescein-Antikörper an die Festphase gebunden werden, oder der Antikörper kann über lmmunglobulin-bindende Proteine an die Festphase oder das Label gebunden werden.

Die erfindungsgemäßen Antikörper eignen sich zur Verwendung in Verfahren zur quantitativen oder qualitativen Bestimmung des Prothrombin-Fragments F2/F1+2 in einer biologischen Probe eines Probanden, bevorzugt in einer Blut- oder Plasmaprobe eines Patienten. Ein weiterer Gegenstand der vorliegenden Erfindung ist daher ein Verfahren zur Bestimmung des Prothrombin-Fragments F2/F1+2 in einer Probe, wobei die Probe mit einem Antikörperfragment mit Spezifität für das carboxyterminale Neoepitop des Prothrombin-Fragmentes F2/F1+2 zur Bildung eines Immunkomplexes mit dem in der Probe enthaltenen Prothrombin-Fragment F2/F1+2 in Kontakt gebracht wird. Weiterhin wird die Probe mit einem erfindungsgemäßen Antikörper oder einem Fragment desselben, der an den Immunkomplex bindet, aber nicht an das Prothrombin-Fragment F2/F1+2 alleine und nicht an das Antikörperfragment mit Spezifität für das carboxyterminale Neoepitop des Prothrombin-Fragmentes F2/F1+2 alleine, in Kontakt gebracht, und die Menge des gebundenen Immunkomplexes wird bestimmt. In einer bevorzugten Ausführungsform wird die Probe mit einem F(ab')2- oder mit einem Fab-Fragment des Antikörpers, der von der hinterlegten Hybridomazelllinie DSM ACC2911 gebildet wird, zur Bildung eines Immunkomplexes mit dem in der Probe enthaltenen Prothrombin-Fragment F2/F1+2 in Kontakt gebracht, und die Menge des Immunkomplexes wird mit Hilfe eines Antikörpers mit Spezifität für diesen Immunkomplex bestimmt, bevorzugt mit Hilfe eines Antikörpers, der von einer der hinterlegten Hybridomazelllinien DSM ACC2912, DSM ACC2913 oder DSM ACC2914 gebildet wird oder eines Fragments derselben.

Bei einem Verfahren zur quantitativen Bestimmung von F2/F1+2 wird die Menge oder die Konzentration von F2/F1+2 in der Probe gemessen. Von dem Begriff des "quantitativen Nachweises" sind auch semiquantitative Methoden umfasst, die nur die ungefähre Menge oder Konzentration von F2/F1+2 in der Probe erfassen oder nur zu einer relativen Mengen- oder Konzentrationsangabe dienen können. Unter einem qualitativen Nachweis ist der Nachweis des Vorhandenseins von F2/F1+2 in der Probe überhaupt oder das Anzeigen, dass die Konzentration von F2/F1+2 in der Probe unterhalb oder oberhalb eines bestimmten oder mehrerer bestimmter Schwellenwerte liegt, zu verstehen.

Die Erfindung betrifft somit auch Verfahren zum quantitativen oder qualitativen Nachweis von F2/F1+2 in einer Probe und geeignete Reagenzien hierfür. Bei diesen Verfahren kann es sich um sogenannte heterogene oder homogene Bindungsteste handeln, bei denen durch eine spezifische Bindung von F2/F1+2 an einen Bindungspartner auf die Anwesenheit, Abwesenheit oder Menge von F2/F1+2 in einer Probe geschlossen werden kann. Immunoassays sind Beispiele für Bindungsteste.

Die sogenannten "heterogenen Bindungsteste" sind durch einen oder mehrere Trennungsschritte und/oder Waschschritte gekennzeichnet. Die Trennung kann beispielsweise durch Immunfällung, Fällung mit Substanzen wie Polyethylenglykol oder Ammoniumsulfat, Filtration, magnetische Abtrennung, Anbindung an eine Festphase erfolgen. Ein solche "Festphase" besteht aus porösem und/oder nicht porösem, in der Regel wasserunlöslichem Material. Sie kann die unterschiedlichsten Formen aufweisen wie z. B.: Gefäß, Röhrchen, Mikrotitrationsplatte, Kugel, Mikropartikel, Stäbchen, Streifen, Filter- oder Chromatographiepapier etc. Bei heterogenen Bindungstesten im Sandwichformat ist in der Regel einer der F2/F1+2-spezifischen Bindungspartner an eine Festphase gebunden und dient zur Abtrennung des Bindungskomplexes "F2/F1+2 - F2/F1+2-spezifischer Bindungspartner" von der flüssigen Phase, während der andere analytspezifische Bindungspartnerzum Nachweis des Bindungskomplexes ein nachweisbares Label (z. B. ein Enzym, ein Fluoreszenz- oder Chemilumineszenzlabel etc.) trägt. Man unterteilt diese Testverfahren weiter in sogenannte Einschritt-Sandwich-Teste, bei denen die beiden spezifischen Bindungspartner simultan mit der Probe inkubiert werden und in Zweischritt-Sandwich-Teste, bei denen die Probe zunächst mit dem Festphasenreagenz inkubiert wird und nach einem Trenn- und Waschschritt der festphasengebundene Bindungskomplex aus F2/F1+2 und F2/F1+2-spezifischem Bindungspartner mit dem Nachweisreagenz inkubiert wird.

Bei "homogenen Bindungstesten" erfolgt keine Trennung zwischen freien und an den "F2/F1+2 - F2/F1+2-spezifischer Bindungspartner"-Komplex gebundenen Komponenten des signalbildenden Systems. Der Testansatz, welcher die F2/F1+2-spezifischen Bindungspartner, die signalbildenden Komponenten und die Probe enthält, wird nach oder sogar während der Bindungsreaktion ohne einen weiteren Trenn- und/oder Waschschritt gemessen und das entsprechende Messsignal bestimmt. Beispiele für homogene Immunoassays sind viele turbidimetrische oder nephelometrische Methoden, wobei die zum Nachweis verwendeten analytspezifischen Bindungspartner mit Latexpartikel assoziiert sein können; EMIT^{®}-Teste; CEDIA^{®}-Teste; Fluoreszenz-Polarisations-Immunoassays; Luminescent Oxygen Channeling Immunoassays ("LOCI", siehe EP 515 194 A2; Ullman, E. F. et al. (1994) Proc. Natl. Acad. Sci., 91: 5426-5430; Ullman, E. F. et al. (1996) Clinical Chemistry ,42: 1518-1526) etc. Bei einem homogenen Sandwich-Immunoassay, wie z. B. einem nephelometrischen Latextest, werden die Antikörperreagenzien mit der Probe zusammen inkubiert und die Messung des Signals während und/oder nach der Inkubation durchgeführt, ohne dass ein Trenn-oder Waschschritt vor der Messung durchgeführt wird. Mit anderen Worten ausgedrückt: Es folgt keine Trennung des Antikörper-gebundenen Analyten vom freien Analyten oder von Antikörpern, die keinen Analyten gebunden haben.

Homogene und heterogene Bindungsteste können auch in Form eines sogenannten "Sandwichassays" durchgeführt werden. Hierbei wird der Analyt z. B. bei einem heterogenen Bindungstest von einem Festphasen-assoziierten analytspezifschen Bindungspartner und einem analytspezifischen Bindungspartner, der mit einer Komponente eines signalbildenden System assoziiert ist, gebunden.

Bei einem homogenen oder heterogenen "kompetitiven Bindungstest" konkurrieren Proben-Analyt und Reagenz-Analyt (beispielsweise ein "modifizierter Analyt" wie z. B. ein gelabeltes oder markiertes F2/F1+2 oder F2/F1+2-Fragment), um die Bindung an eine limitierte Anzahl von analytspezifischen Bindungspartnern. Beispiele zur Illustration des Prinzips: (i) Proben-Analyt konkurriert mit Reagenz-Analyt, der mit einer Komponente eines signalbildenden System assoziiert ist, um die Bindung an Festphasen-assoziierte analytspezifische Bindungspartner oder (ii) Proben-Analyt konkurriert mit Festphasen-assoziiertem Analyt (= Reagenz-Analyt) um die Bindung an analytspezifische Bindungspartner, die mit einer Komponente eines signalbildenden Systems assoziiert sind.

Der Nachweis von F2/F1+2 mit den erfindungsgemäßen Antikörpern kann beispielsweise auch mit Verfahren erfolgen wie beispielsweise: Western Blot, Dot Blot, Immunelektrophorese, Immunfixations-Elektrophorese, Elektroimmundiffusion, Immunpräzipitation, radiale Immundiffusion, Immunfixation, Immunchromatographie, Latex-Agglutination, turbidimetrischer oder nephelometrischer Test, homogener oder heterogener Bindungstest, Ein- oder Zweischritt-Test, Sandwich-Test, indirekter Test, kompetitiver Test, "point-of-care"-Teste etc. Diese und andere Nachweisverfahren sind beispielsweise in "Labor und Diagnose", ed. L. Thomas, TH-Books Verlagsgesellschaft mbH, Frankfurt, 1998, Kapitel 60 beschrieben.

Der Begriff "point-of-care-Teste" oder "POC-Teste" schließt Teste ein, bei denen kein separates Analyse- oder Messgerät zur Testdurchführung oder Testauswertung benötigt wird. POC-Teste basieren in vielen Fällen auf immunchromatographischen Verfahren, Immunkomplexabtrennungen- per Filtration und/oder Immunfixationstechniken. Die POC-Teste sind insbesondere für Messungen vor Ort, z. B. am Krankenbett oder daheim, für den Notarzt und/oder beim niedergelassenen Arzt und weniger für das Großlabor gedacht. POC-Teste können insbesondere auch von Personen, die keine eingehende medizinischtechnische Ausbildung und Erfahrung auf dem Gebiet der Laboratoriumsmedizin haben, durchgeführt werden. Unter der Begriff "POC-Teste" sind im Sinne dieser Erfindung auch sogenannte Heimteste oder OTC-Teste zu verstehen, die von medizinischen Laien durchgeführt werden dürfen, so z. B. die diversen Schwangerschaftsteste die für den Heimgebrauch vertrieben werden. Andere POC-Teste betreffen beispielsweise den Nachweis von Herzinfarktmarkern, Drogen, Arzneimitteln, Infektions- und Entzündungsmarkern. Bei vielen POC-Testen sind oder werden im Laufe der Testdurchführung spezifische Bindungspartner an oder auf Filter- oder Chromatographiestreifen oder -scheiben assoziiert. Eine positive oder negative Nachweisreaktion kann zum Beispiel mit dem Erscheinen oder Nichterscheinen einer Farbbande in einem bestimmten Testfeld verknüpft sein und/oder dem Erscheinen oder Nichterscheinen eines bestimmten Symbols, z.B. einem "+",einem "-" und/oder der Intensität des jeweiligen Meßsignals.

Ein POC-Test für F2/F1+2 kann beispielsweise so aufgebaut sein: Probe und gelabelte Antikörperfragmente mit Spezifität für das carboxyterminale Neoepitop des Prothrombin-Fragmentes F2/F1+2 werden auf einen Teststreifen aufgetragen. Geeignete Label sind z. B. gefärbte Latexpartikel, kolloidales Gold, Enzyme etc. Sofern F2/F1+2 in der Probe enthalten ist, werden sich F2/F1+2-Antikörperfragment-Komplexe ausbilden. Diese Komplexe bewegen sich z. B. mittels Kapillarkraft in Richtung auf einen Bereich, in dem erfindungsgemäße Antikörper, die spezifisch an den Immunkomplex enthaltend F2/F1+2 und das Antikörperfragment mit Spezifität für das carboxyterminale Neoepitop des Prothrombin-Fragmentes F2/F1+2 zu binden vermögen, z. B. in Form einer Bande fixiert sind oder im Laufe des Testverfahrens fixiert werden (z. B. über eine Biotin-Avidin-Brücke). Die gelabelten F2/F1+2-Antikörper-Immunkomplexe werden in diesem Bereich gebunden und bilden mit den fixierten Antikörpern einen Sandwichkomplex aus. Die Intensität des Labelsignals ist hier proportional zur F2/F1+2-Probenkonzentration.

Ein anderer erfindungsgemäßer Gegenstand ist ein Testkit, der mindestens ein Reagenz enthält, das einen erfindungsgemäßen Antikörper enthält, der an einen Immunkomplex enthaltend Prothrombin-Fragment F2, an welches ein Antikörperfragment mit Spezifität für das carboxyterminale Neoepitop des Prothrombin-Fragmentes F2/F1+2 gebunden ist, bindet, aber nicht an das Prothrombin-Fragment F2 alleine und nicht an das Antikörperfragment mit Spezifität für das carboxyterminale Neoepitop des Prothrombin-Fragmentes F2/F1+2 alleine. Ein bevorzugtes Testkit enthält einen Antikörper, der von einer der hinterlegten Hybridomazelllinien DSM ACC2912, DSM ACC2913 oder DSM ACC2914 gebildet wird. In einem solchen Kit sind üblicherweise alle oder nur einige Komponenten eines Testes in abgepackter Form enthalten. Die erfindungsgemäßen Antikörper können beispielsweise mit einer oder mehreren Festphasen und/oder einer oder mehreren Komponenten eines signalbildenden Systems assoziiert sein. Das Testkit kann beispielsweise Standards, Kontrollen, sowie andere Reagenzien, wie z. B. Puffer, Waschlösungen, Messsignalauslösende Lösungen und/oder Enzymsubstrat; Küvetten; Pipetten und/oder Testanweisungen enthalten.

Bevorzugterweise enthält ein erfindungsgemäßer Testkit weiterhin einen Antikörper oder ein Antikörperfragment mit Spezifität für das carboxyterminale Neoepitop des Prothrombin-Fragmentes F2/F1+2, bevorzugterweise ein F(ab')2-oder ein Fab-Fragment des monoklonalen Antikörpers 96-163/04, der von der hinterlegten Hybridomazelllinie DSM ACC2911 gebildet wird.

### Figur 1

Kalibrationskurven für die Quantifizierung von F2/F1+2 in einem homogenen LOCI®-Assay (siehe Ausführungsbeispiel 3). Die gemessenen Signale verhalten sich sowohl in einem ersten Testaufbau, bei dem biotinylierte Immunkomplexspezifische Antikörper (BA) in Kombination mit Chemibead-gekoppeltem F27F1+2-Neoepitop-spezifischem Antikörper (CB) eingesetzt wurden (durchgezogene Linien) als auch in einem zweiten Testaufbau, bei dem ein biotinylierter F2/F1+2-Neoepitop-spezifischer Antikörper (BA) in Kombination mit Chemibeadgekoppelten Immunkomplex-spezifischen Antikörpern (CB) eingesetzt wurde (gestrichelte Linien), proportional zur F1+2-Konzentration.

Die folgenden Ausführungsbeispiele dienen der Veranschaulichung der vorliegenden Erfindung und sind nicht als Einschränkung zu verstehen.

### Beispiel 1: Herstellung verschiedener Immunkomplexe zur Verwendung als Immunisierungantigene

Folgende Immunkomplexe wurden als Immunisierungsantigene verwendet:

**Tabelle 1**

| | **Immunkomplex** | | | |
|---|---|---|---|---|
| | **Prothrombinfragment** | **F2/F1+2-Antikörper 96-163/04** | **Quervernetzung** | **Trägerprotein** |
| a) | F2/F1+2-Peptid 15mer | komplett | --- | --- |
| b) | F2/F1+2-Peptid 15mer | komplett | Glutaraldehyd | --- |
| c) | vollständiges F2-Fragment | komplett | --- | --- |
| d) | vollständiges F2-Fragment | komplett | Glutaraldehyd | --- |
| e) | vollständiges F2-Fragment | Fab-Fragment | Glutaraldehyd | KLH |
| f) | vollständiges F2-Fragment | Fab-Fragment | --- | Ovalbumin |

### Ausgangsmaterialien:

### Verwendete Prothrombinfragmente:

### F2/F1+2-Peptid 15mer

Synthetisches Peptid bestehend aus den 14 C-terminalen Aminosäureresten des humanen Prothrombinfragments F2/F1+2 und einem zusätzlichen aminoterminalen Cystein-Rest (Sequenz: H-Cys-Leu-Asp-Glu-Asp-Ser-Asp-Glu-Glu-Arg-Ala-Ile-Glu-Gly-Arg-OH).

### vollständiges F2-Fragment

Zur Gewinnung eines vollständigen F2-Fragments (14 kDa) wurde ein kommerziell erhältliches, Gerinnungsfaktor-Konzentrat (humaner Prothrombinkomplex) als Prothrombinquelle verwendet (Beriplex P/N 500, CSL Behring GmbH, Marburg, Deutschland). Die Herstellung der F2-Präparation wurde folgendermaßen durchgeführt:
1. Lösen von Beriplex in Aktivierungspuffer (10 mmol Tris, 10 mmol CaCl₂, pH 7,5),
2. Gerinnungsaktivierung mittels FX-spezifischer RVV-Sepharose (Schlangengift gekoppelt auf Trägermaterial) und Inkubation unter Rühren bei 37 °C,
3. Abtrennung des Trägermaterials mittels Nutsche oder Zentrifugation und anschließendes Abstoppen der Gerinnungsaktivität mittels PMSF (Phenylmethylsulfonylfluorid) sowie Zugabe von Natrium-Citrat,
4. Zweimalige Adsorption/Ausfällung der Spaltprodukte mit Gla-Domänen mittels Bariumchlorid und Abtrennung mittels Zentrifugation,
5. Stabilisierung, Filtration und Ankonzentrierung des Überstandes,
6. Gelfiltration des Überstandes mittels Superdex^{™} 75 Gelmaterial (GE Healthcare Europe GmbH, Deutschland), Laufpuffer 50 mmol NaHPO₄, pH 8,5,
   Verwendung der F2-haltigen Fraktionen für die weitere Aufreinigung,
7. Anionenaustauscher-Chromatographie unter Verwendung von Mono Q
   Trägermaterial (GE Healthcare Europe GmbH, Deutschland):
      Puffer A: 50 mmol NaHPO₄, pH 8,5
      Puffer B: 50 mmol NaHPO₄ + 1 mol NaCl, pH 8,5
      Auftrag der F2-haltigen Fraktionen in Puffer A, Elution mittels linearem
      Salzgradienten (0-40 % Puffer B, 20 Säulenvolumen), Sammeln und Poolen der F2-haltigen Fraktionen,
8. Überprüfung der Reinheit der Präparation mittels SDS-PAGE und Western-Blot.

### Verwendete Antikörper bzw. Antikörperfragmente:

Der monoklonale Antikörper F1+2 96-163/04 mit Spezifität für das carboxyterminale Neoepitop des Prothrombin-Fragmentes F2/F1+2 wurde gemäß der Lehre in US 5,830,681 (Hursting et al.) hergestellt. Die den Antikörper produzierende Hybridomazelllinie wurde unter der Eingangsnummer DSM ACC2911 hinterlegt. Für die Herstellung des Fab-Fragments wurde der Antikörper F1+2 96-163/04 mittels bekannter Verfahren mit Papain gespalten und das Fab-Fragment wurde angereichert.

### Herstellung der Immunkomplexe:

Zur Herstellung der Immunkomplexe wurde das synthetische F2/F1+2-Peptid (15mer) bzw. das gereinigte vollständige F2-Fragment in 10-30 molarem Überschuss zum dem monoklonalen Antikörper F1+2 96-163/04 bzw. zu dessen Fab-Fragment gegeben, in PBS-Puffer (pH 7,2) für 3 bis 3,5 Stunden bei Raumtemperatur inkubiert und anschließend wurde der Immunkomplex über Sephacryl^{™} S-100 (GE Healthcare Europe GmbH, Deutschland) chromatographisch aufgereinigt.

### Quervernetzung von Immunkomplexen:

Glutaraldehyd wurde in einer Konzentration von 0,2 % zum Ansatz gegeben, und der Ansatz wurde 2 Stunden bei 2-8 °C inkubiert. Die Reaktion wurde durch Zugabe von NaBH₄ abgestoppt, und der vernetzte Immunkomplex wurde über eine HiPrep^{™}-Desalting Säule (GE Healthcare Europe GmbH, Deutschland) aufgereinigt (Laufpuffer PBS pH 7,2).

### Kopplung an Trägerproteine:

Die Kopplung der Immunkomplexe bzw. der quervernetzten Immunkomplexe an Keyhole Limpet Hämocyanin (KLH) oder Ovalbumin erfolgte gemäß bekannter Routineverfahren.

### Beispiel 2: Herstellung und Screening erfindungsgemäßer Antikörper

Gemäß dem Stand der Technik wurden BALB/c Mäuse jeweils mit 20 µg Immunisierungsantigen (siehe Immunkomplexe aus Beispiel 1) intraperitoneal immunisiert. Milzzellen der immunisierten Tiere wurden mit Myelomzellen (Sp2/0) fusioniert, und es wurden Hybrodimazelllinien etabliert.

### Geeignete Antikörper wurden wie folgt identifiziert:

### ELISA Nr. 1: Reaktivität mit dem Immunkomplex bestehend aus F2-Fragment und dem F(ab')2-Fragment des monoklonalen Antikörpers F1+2-96-163/04

Mit dem F(ab')2-Fragment des monoklonalen Antikörpers F1+2 96-163/04 beschichtete Mikrotiterplatten wurden mit einer Lösung enthaltend F2-Fragment (siehe Beispiel 1) inkubiert, um einen Immunkomplex aus dem F(ab')2-Fragment des F2/F1+2-spezifischen Antikörpers und dem F2-Fragment zu bilden.

### ELISA Nr. 2: Reaktivität mit dem F2-Fragment allein

Mikrotiterplatten wurden mit gereinigtem F2-Fragment (siehe Beispiel 1) beschichtet.

### ELISA Nr. 3: Reaktivität mit dem F(ab')2 Fragment des monoklonalen Antikörpers F1+2 96-163/04 allein

Mikrotiterplatten wurden mit dem F(ab')2-Fragment des F2/F1+2-spezifischen Antikörpers 96-163/04 beschichtet.

In jede Vertiefung der beschichteten Mikrotiterplatten wurden 100 µl Zellkulturüberstand (Verdünnung 1:2) pipettiert und 1 Stunde bei +15 bis +25 °C inkubiert. Nach zweimaligen Waschen der Platte mit Waschlösung-POD (OSEW; Siemens Healthcare Diagnostics Products GmbH, Marburg, Deutschland) wurden in jede Vertiefung 100 µl anti-Maus lgG/Fc-POD-Konjugat (Peroxidase-Konjugat, Sigma-Aldrich GmbH, Deutschland, Prod. Nr. A0168) eingefüllt und 1 Stunde bei +15 bis +25 °C inkubiert. Nach weiterem zweimaligen Waschen der Platte wurde in jede Vertiefung 100 µl Chromogen TMB-Lösung (Tetramethylbenzidin-Lösung, Siemens Healthcare Diagnostics Products GmbH, Marburg, Deutschland) eingefüllt und weitere 30 Minuten bei +15 bis +25 °C inkubiert. Nach der Inkubation wurde in jede Vertiefung 100 µl Stopplösung POD (Siemens Healthcare Diagnostics Products GmbH, Marburg, Deutschland) eingefüllt und die Mikrotiterplatte am Tecan Sunrise^{™} Absorbance Reader (Tecan Deutschland, GmbH, Crailsheim, Deutschland) bei 450 nm ausgewertet.

Die Zelllinien, deren Zellkulturüberstände eine gute Reaktivität mit dem Immunkomplex (ELISA Nr. 1) und eine minimale bis keine Reaktivität mit den einzelnen Komponenten zeigten (ELISA Nr. 2 und 3), wurden kloniert, und aus diesen Klonen wurden größere Mengen Antikörper gereinigt. Die Konzentration der gereinigten Antikörper wurde auf 1 bzw 0,1 µg/mL eingestellt und in den ELISA's Nr. 1 bis 3 gestestet.

Die Ergebnisse sind in den Tabellen 2 (Antikörperkonzentration 1 µg/mL) und 3 (Antikörperkonzentration 0,1 µg/mL ) dargestellt. Als Messdaten sind die Extinktionswerte bei 450 nm angegeben.

**Tabelle 2**

| **MAK** | **Gruppe Nr.** | **ELISA Nr. 1** | **ELISA Nr. 2** | **ELISA Nr. 3** |
|---|---|---|---|---|
| **2006-140/0651** | 1 | 3,961 | 0,038 | 0,511 |
| **2006-175/024** | 1 | 2,761 | 0,028 | 0,108 |
| **2006-188/044** | 1 | 3,906 | 0,027 | 0,186 |
| **2006-188/069** | 1 | 3,848 | 0,029 | 0,233 |
| **2006-188/086** | 1 | 3,918 | 0,036 | 0,174 |
| **2006-188/0143** | 1 | 3,970 | 0,031 | 0,387 |
| **2006-209/09** | 1 | 3,819 | 0,030 | 0,166 |
| **2006-216/04** | 1 | 1,940 | 0,024 | 0,230 |
| **Prothr.195/097** | 2 | 4,103 | 3,570 | 0,135 |
| **96-163/04** | 3 | 0,923 | 1,921 | 0,112 |
| **95-332/03** | 3 | 0,290 | 3,552 | 0,097 |
| **CRP 268/094** | 4 | 0,062 | 0,027 | 0,099 |
| **Background** | - | 0,082 | 0,034 | 0,097 |

**Tabelle 3**

| **MAK** | **Gruppe Nr.** | **ELISA Nr. 1** | **ELISA Nr. 2** | **ELISA Nr. 3** |
|---|---|---|---|---|
| **2006-140/0651** | 1 | 3,645 | 0,025 | 0,135 |
| **2006-175/024** | 1 | 1,490 | 0,034 | 0,107 |
| **2006-188/044** | 1 | 3,808 | 0,034 | 0,099 |
| **2006-188/069** | 1 | 3,779 | 0,031 | 0,121 |
| **2006-188/086** | 1 | 3,835 | 0,024 | 0,107 |
| **2006-188/0143** | 1 | 3,795 | 0,025 | 0,132 |
| **2006-209/09** | 1 | 3,171 | 0,027 | 0,110 |
| **2006-216/04** | 1 | 1,548 | 0,020 | 0,102 |
| **Prothr. 195/097** | 2 | 3,534 | 1,850 | 0,102 |
| **96-163/04** | 3 | 0,174 | 0,615 | 0,096 |
| **95-332/03** | 3 | 0,096 | 1,203 | 0,096 |
| **CRP 268/094** | 4 | 0,081 | 0,025 | 0,101 |
| **Background** | - | 0,082 | 0,034 | 0,097 |

Die getesteten monoklonalen Antikörper wurden für eine bessere Übersichtlichkeit in Gruppen aufgeteilt:
Gruppe 1: erfindungsgemäße Antikörper mit Spezifität für den Immunkomplex bestehend aus F2-Fragment und dem F(ab')2-Fragment des F2/F1+2-Neoepitop-spezifischen Antikörpers F1+2 96-163/04;
Gruppe 2: Prothrombin-spezifischer Antikörper, der an ein Epitop auf der N-terminalen Hälfte des F2-Fragments bindet (siehe EP 1 541 676 A1, Beispiel 2 d) und e), Hybridomazelllinie unter Eingangsnummer DSM ACC2607 hinterlegt), mitgeführt als Kontrolle;
Gruppe 3: Monoklonale Antikörper mit Spezifität für das carboxyterminale Neoepitop des Prothrombin-Fragmentes F2/F1+2, hergestellt gemäß der Lehre in US 5,830,681 (Hursting et al.), mitgeführt als Kontrolle;
Gruppe 4: systemfremder monoklonaler Antikörper, mitgeführt als Negativkontrolle.

Die erfindungsgemäßen Antikörper der Gruppe 1 zeigen die gewünschten Eigenschaften eines gegen den lmmunkomplex gerichteten monoklonalen Antikörpers, nämlich im ELISA Nr. 1 eine möglichst gute Reaktivität mit dem Komplex, im ELISA Nr. 2 und 3 hingegen nur minimale bis keine Reaktion.

### Beispiel 3: Homogener LOCI®-Assay zur Quantifizierung von F2/F1+2

Die hier verwendete LOCI® Technologie beruht darauf, dass eine auf Latexpartikein gekoppelte Chemilumineszenzverbindung (Chemibeads, CB) und ein auf Latexpartikeln gekoppelter Photosensibilisator (Sensibeads, SB) durch Bindung an einen Analyten in eine räumliche Nähe zueinander gebracht werden, so dass Singulett-Sauerstoff, der vom Photosensibilisator erzeugt wird, die Chemilumineszenzverbindung anregen kann. Die Menge der erzeugten Chemilumineszenz korreliert mit der Analytmenge. Unter Verwendung der LOCI® Technologie wurde eine Signalabhängigkeit von der F2/F1+2-Konzentration in einem homogenen immunologischen Testaufbau gezeigt.

Für einen ersten Testaufbau wurden die Immunkomplex-spezifischen Antikörper 2006-175/024 (aus DSM ACC 2912), 2006-188/044 (aus DSM ACC 2913) oder 2006-188/069 (aus DSM ACC 2914) auf Chemibeads gekoppelt. Als zweite Komponente wurden Streptavidin-beschichtete Sensibeads eingesetzt. Als dritte Komponente wurde der biotinylierte F2/F1+2-Neoepitop-spezifische Antikörper 96-163/04 (aus DSM ACC2911) eingesetzt.

Für einen zweiten Testaufbau wurde der F2/F1+2-Neoepitop-spezifische Antikörper 96-163/04 (aus DSM ACC2911) auf Chemibeads gekoppelt. Als zweite Komponente wurden Streptavidin-beschichtete Sensibeads eingesetzt. Als dritte Komponente wurden die biotinylierten Immunkomplex-spezifischen Antikörper 2006-175/024 (aus DSM ACC 2912), 2006-188/044 (aus DSM ACC 2913) oder 2006-188/069 (aus DSM ACC 2914) eingesetzt.

Zur Quantifizierung der F2/F1+2-Konzentration wurde eine Plasmaprobe mit den beiden Reagenzien (Chemibeads und biotinyliertem Antikörper) gemischt und für 220 Sekunden bei 37 °C inkubiert. Danach wurden die Sensibeads als weitere Signalkomponente zugegeben und gemischt. Hiernach erfolgte eine erneute Inkubation über 360 Sekunden bei 37 °C. Im Anschluss erfolgte die Messung der Chemilumineszenz im LOCI-Reader.

Zur Quantifizierung der F2/F1+2-Konzentration in Humanplasmaproben wurde eine Standardkurve erstellt. Hierfür wurde F1+2 in einem geeigneten Puffer auf eine definierte Konzentration von 5000 pmol/l eingestellt. Von dieser Stammlösung wurde eine Verdünnungsreihe angelegt und wie oben beschrieben analysiert. Die Auftragung der F1+2-Konzentration (X-Achse) gegen die gemessenen Signalhöhen ergibt eine Kalibrationskurve. Wie aus Figur 1 ersichtlich, ist das generierte Signal in den beiden verschiedenen Testaufbauten proportional der F1+2 Konzentration in der Probe, welches die Quantifizierung der F2/F1-+2 Konzentration ermöglicht.

Weitere Untersuchungen haben gezeigt, dass mit den hier gewählten Testaufbauten kein High-Dose-Hook-Effekt bis zu einer Konzentration von 1600000 pmol/l F1+2 festgestellt wurde. Eine falsch niedrige Bestimmung des Analyten ist somit nahezu ausgeschlossen.

## Patentansprüche

1. Isolierter Immunkomplex enthaltend ein Peptid, das zumindest das vollständige Prothrombin-Fragment F2 umfasst, an dessen Carboxyterminus ein Antikörperfragment mit Spezifität für das carboxyterminale Neoepitop des Prothrombin-Fragmentes F2/F1+2, welches zumindest die vier carboxyterminalen Aminosäuren Ile-Glu-Gly-Arg-OH enthält, gebunden ist, wobei der Immunkomplex an ein immunstimulatorisches Trägerprotein gekoppelt ist.

2. Immunkomplex gemäß Anspruch 1, wobei das gebundene Antikörperfragment ein Fab, Fab', F(ab')2 oder Fv-Fragment ist.

3. Immunkomplex gemäß einem der vorhergehenden Ansprüche, wobei das gebundene Antikörperfragment ein Fragment eines monoklonalen Antikörpers ist, der von der Hybridomazelllinie DSM ACC 2911 gebildet wird.

4. Immunkomplex gemäß einem der vorhergehenden Ansprüche, der an das immunstimulatorische Trägerprotein Keyhole Limpet Hämocyanin gekoppelt ist.

5. Immunkomplex gemäß einem der vorhergehenden Ansprüche, der mittels eines Aldehyds quervernetzt ist.

6. Verwendung eines Immunkomplexes gemäß einem der Ansprüche 1 bis 5 als Immunisierungsantigen in einem Verfahren zur Herstellung eines monoklonalen Antikörpers.

7. Monoklonaler Antikörper **dadurch gekennzeichnet, dass** er an einen Immunkomplex bindet, wobei der Immunkomplex ein Peptid enthält, das zumindest das vollständige Prothrombin-Fragment F2 umfasst und an dessen Carboxyterminus ein Antikörperfragment mit Spezifität für das carboxyterminale Neoepitop des Prothrombin-Fragmentes F2/F1+2, welches zumindest die vier carboxyterminalen Aminosäuren Ile-Glu-Gly-Arg-OH enthält, gebunden ist, wobei der monoklonale Antikörper aber nicht an das Prothrombin-Fragment F2 alleine und nicht an das Antikörperfragment mit Spezifität für das carboxyterminale Neoepitop des Prothrombin-Fragmentes F2/F1+2 alleine bindet.

8. Monoklonaler Antikörper gemäß Anspruch 7 **dadurch gekennzeichnet, dass** dieser von einer der Hybridomazelllinien DSM ACC2912, DSM ACC2913 oder DSM ACC2914 produziert wird.

9. Monoklonaler Antikörper gemäß einem der Ansprüche 7 und 8 **dadurch gekennzeichnet, dass** er mit einer Komponente eines signalgebenden Systems assoziiert ist.

10. Monoklonaler Antikörper gemäß einem der Ansprüche 7 bis 9 **dadurch gekennzeichnet, dass** er mit einer Festphase assoziiert ist.

11. Verwendung eines monoklonalen Antikörpers gemäß einem der Ansprüche 7 bis 10 oder eines Fragments desselben, welches an einen Immunkomplex bindet, wobei der Immunkomplex ein Peptid enthält, das zumindest das vollständige Prothrombin-Fragment F2 umfasst und an dessen Carboxyterminus ein Antikörperfragment mit Spezifität für das carboxyterminale Neoepitop des Prothrombin-Fragmentes F2/F1+2, welches zumindest die vier carboxyterminalen Aminosäuren Ile-Glu-Gly-Arg-OH enthält, gebunden ist, wobei der monoklonale Antikörper aber nicht an das Prothrombin-Fragment F2 alleine und nicht an das Antikörperfragment mit Spezifität für das carboxyterminale Neoepitop des Prothrombin-Fragmentes F2/F1+2 alleine bindet, in einem in vitro Verfahren zum quantitativen oder qualitativen Nachweis von F2/F1+2.

12. Hybridomazelllinie, die einen monoklonalen Antikörper gemäß Anspruch 7 produziert.

13. Hybridomazelllinie gemäß Anspruch 12, die bei der DSMZ unter der Eingangsnummer DSM ACC2912, DSM ACC2913 oder DSM ACC2914 hinterlegt ist.

14. Reagenz enthaltend einen monoklonalen Antikörper gemäß einem der Ansprüche 7 bis 10 oder ein Fragment desselben, welches an einen Immunkomplex bindet, wobei der Immunkomplex ein Peptid enthält, das zumindest das vollständige Prothrombin-Fragment F2 umfasst und an dessen Carboxyterminus ein Antikörperfragment mit Spezifität für das carboxyterminale Neoepitop des Prothrombin-Fragmentes F2/F1+2, welches zumindest die vier carboxyterminalen Aminosäuren Ile-Glu-Gly-Arg-OH enthält, gebunden ist, wobei der monoklonale Antikörper aber nicht an das Prothrombin-Fragment F2 alleine und nicht an das Antikörperfragment mit Spezifität für das carboxyterminale Neoepitop des Prothrombin-Fragmentes F2/F1+2 alleine bindet.

15. Testkit enthaltend ein Reagenz gemäß Anspruch 14.

16. Verfahren zur Bestimmung des Prothrombin-Fragments F2/F1+2 in einer Probe, wobei die Probe mit einem Antikörper oder einem Antikörperfragment mit Spezifität für das carboxyterminale Neoepitop des Prothrombin-Fragmentes F2/F1+2 zur Bildung eines Immunkomplexes mit dem in der Probe enthaltenen Prothrombin-Fragment F2/F1+2 in Kontakt gebracht wird **dadurch gekennzeichnet, dass** die Probe zusätzlich mit einem Antikörper gemäß Anspruch 7 in Kontakt gebracht wird.

## Claims

1. An isolated immune complex comprising a peptide which includes at least the complete prothrombin fragment F2 to whose carboxy terminus an antibody fragment with specificity for the carboxy-terminal neoepitope of the prothrombin fragment F2/F1+2, which comprises at least the four carboxy-terminal amino acids Ile-Glu-Gly-Arg-OH, is bound, wherein the immune complex is coupled to an immunostimulatory carrier protein.

2. An immune complex as claimed in claim 1, where the bound antibody fragment is an Fab, Fab', F(ab')₂ or Fv fragment.

3. An immune complex as claimed in either of the preceding claims, where the bound antibody fragment is a fragment of a monoclonal antibody formed by the hybridoma cell line DSM ACC 2911.

4. An immune complex as claimed in any of the preceding claims, which is coupled to the immunostimulatory carrier protein keyhole limpet hemocyanin.

5. An immune complex as claimed in any of the preceding claims, which is crosslinked by means of an aldehyde.

6. The use of an immune complex as claimed in any of claims 1 to 5 as immunizing antigen in a method for preparing a monoclonal antibody.

7. A monoclonal antibody which binds to an immune complex where the immune complex contains a peptide which comprises at least the complete prothrombin fragment F2 to whose carboxy terminus an antibody fragment with specificity for the carboxy-terminal neoepitope of the prothrombin fragment F2/F1+2, which comprises at least the four carboxy-terminal amino acids Ile-Glu-Gly-Arg-OH, is bound, wherein the monoclonal antibody does not, however, bind to the prothrombin fragment F2 alone and not to the antibody fragment with specificity for the carboxy-terminal neoepitope of the prothrombin fragment F2/F1+2 alone.

8. A monoclonal antibody as claimed in claim 7, which is produced by one of the hybridoma cell lines DSM ACC2912, DSM ACC2913 or DSM ACC2914.

9. A monoclonal antibody as claimed in either of claims 7 and 8, which is associated with a component of a signal-generating system.

10. A monoclonal antibody as claimed in any of claims 7 to 9, which is associated with a solid phase.

11. The use of a monoclonal antibody as claimed in any of claims 7 to 10 or of a fragment thereof which binds to an immune complex, where the immune complex contains a peptide which comprises at least the complete prothrombin fragment F2 to whose carboxy terminus an antibody fragment with specificity for the carboxy-terminal neoepitope of the prothrombin fragment F2/F1+2, which comprises at least the four carboxy-terminal amino acids Ile-Glu-Gly-Arg-OH, is bound, where, however, the monoclonal antibody does not bind to the prothrombin fragment F2 alone and not to the antibody fragment with specificity for the carboxy-terminal neoepitope of the prothrombin fragment F2/F1+2 alone, in an in-vitro method for the quantitative or qualitative detection of F2/F1+2.

12. A hybridoma cell line which produces a monoclonal antibody as claimed in claim 7.

13. A hybridoma cell line as claimed in claim 12, which is deposited at the DSMZ under the accession number DSM ACC2912, DSM ACC2913 or DSM ACC2914.

14. A reagent comprising a monoclonal antibody as claimed in any of claims 7 to 10 or a fragment thereof which binds to an immune complex, where the immune complex contains a peptide which comprises at least the complete prothrombin fragment F2 to whose carboxy terminus an antibody fragment with specificity for the carboxy-terminal neoepitope of the prothrombin fragment F2/F1+2, which comprises at least the four carboxy-terminal amino acids Ile-Glu-Gly-Arg-OH, is bound, where, however, the monoclonal antibody does not bind to the prothrombin fragment F2 alone and not to the antibody fragment with specificity for the carboxy-terminal neoepitope of the prothrombin fragment F2/F1+2 alone.

15. A test kit comprising a reagent as claimed in claim 14.

16. A method for determining the prothrombin fragment F2/F1+2 in a sample, where the sample is brought into contact with an antibody or an antibody fragment with specificity for the carboxy-terminal neoepitope of the prothrombin fragment F2/F1+2 to form an immune complex with the prothrombin fragment F2/F1+2 present in the sample, wherein the sample is additionally brought into contact with an antibody as claimed in claim 7.

## Revendications

1. Immunocomplexe isolé contenant un peptide qui comprend au moins le fragment de prothrombine complet F2, à la terminaison carboxyle duquel est fixé un fragment d'anticorps ayant de la spécificité pour le néoépitope à terminaison carboxy du fragment de prothrombine F2/F1+2 qui contient au moins les quatre acides aminés à terminaison carboxy lle-Glu-Gly-Arg-OH, l'immunocomplexe étant couplé a une protéine support immunostimulatrice.

2. Immunocomplexe suivant la revendication 1, dans lequel le fragment d'anticorps fixé est un Fab, Fab', F(ab')2 ou Fv.

3. Immunocomplexe suivant l'une des revendications précédentes, dans lequel le fragment d'anticorps fixé est un fragment d'un anticorps monoclonal, qui est formé par la lignée cellulaire d'hybridome DSM ACC 2911.

4. Immunocomplexe suivant l'une des revendications précédentes, qui est couplé à la protéine support immunostimulatrice keyhole limpet hémocyanine.

5. Immunocomplexe suivant l'une des revendications précédentes, qui est réticulé transversalement au moyen d'un aldéhyde.

6. Utilisation d'un immunocomplexe suivant l'une des revendications 1 à 5 comme antigène d'immunisation dans un procédé de préparation d'un anticorps monoclonal.

7. Anticorps monoclonal **caractérisé en ce qu'**il se fixe à un immunocomplexe, l'immunocomplexe contenant un peptide, qui comprend au moins le fragment de prothrombine complet F2 et à la terminaison carboxy duquel est fixé un fragment d'anticorps ayant de la spécificité pour le néoépitope à terminaison carboxy du fragment de prothrombine F2/F1+2, qui contient au moins les quatre acides aminés à terminaison carboxy Ile-Glu-Gly-Arg-OH, mais l'anticorps monoclonal ne se fixant pas au fragment de prothrombine F2 seul et ne se fixant pas au fragment d'anticorps ayant de la spécificité pour le néoépitope à terminaison carboxy du fragment de prothrombine F2/F1+2 seul.

8. Anticorps monoclonal suivant la revendication 7, **caractérisé en ce qu'**il est produit par l'une des lignées cellulaires d'hydridome DSM ACC2912, DSM ACC2913 ou DSM ACC2914.

9. Anticorps monoclonal suivant l'une des revendications 7 et 8, **caractérisé en ce qu'**il est associé à un composant d'un système donnant un signal.

10. Anticorps monoclonal suivant l'une des revendications 7 à 9, **caractérisé en ce qu'**il est associé à une phase solide.

11. Utilisation d'un anticorps monoclonal suivant l'une des revendications 7 à 10 ou de l'un de ses fragments qui se fixe à un immunocomplexe, l'immunocomplexe contenant un peptide, qui comprend au moins le fragment de prothrombine complet F2 et à la terminaison carboxy duquel est fixé un fragment d'anticorps ayant de la spécificité pour le néoépitope à terminaison carboxy du fragment de prothrombine F2/F1+2, qui contient au moins les quatre acides aminés à terminaison carboxy Ile-Glu-Gly-Arg-OH, mais l'anticorps monoclonal ne se fixant pas au fragment de prothrombine F2 seul et ne se fixant pas au fragment d'anticorps ayant de la spécificité pour le néoépitope à terminaison carboxy du fragment de prothrombine F2/F1+2 seul, dans un procédé in vitro de détection quantitative ou qualitative de F2/F1+2.

12. Lignée cellulaire d'hybridome, qui produit un anticorps monoclonal suivant la revendication 7.

13. Lignée cellulaire d'hydridome suivant la revendication 12, qui dans le DSMZ est mémorisée sous le numéro d'entrée DSM ACC2912, DSM ACC2913 ou DSM ACC2914.

14. Réactif contenant un anticorps monoclonal suivant l'une des revendications 7 à 10 ou l'un de ses fragments qui se fixe à un immunocomplexe, l'immunocomplexe contenant un peptide, qui comprend au moins le fragment de prothrombine complet F2 et à la terminaison carboxy duquel est fixé un fragment d'anticorps ayant de la spécificité pour le néoépitope à terminaison carboxy du fragment de prothrombine F2/F1+2, qui contient au moins les quatre acides aminés à terminaison carboxy Ile-Glu-Gly-Arg-OH, mais l'anticorps monoclonal ne se fixant pas au fragment de prothrombine F2 seul et ne se fixant pas au fragment d'anticorps ayant de la spécificité pour le néoépitope à terminaison carboxy du fragment de prothrombine F2/F1+2 seul.

15. Trousse de test contenant un réactif suivant la revendication 14.

16. Procédé de détermination du fragment de prothrombine F2/F1+2 dans un échantillon, l'échantillon étant mis en contact avec un anticorps ou un fragment d'anticorps ayant de la spécificité pour le néoépitope à terminaison carboxy du fragment de prothrombine F2/F1+2 pour la formation d'un immunocomplexe avec le fragment de prothrombine F2/F1+2 contenu dans l'échantillon, **caractérisé en ce que** l'on met l'échantillon supplémentairement en contact avec un anticorps suivant la revendication 7.
